# EUROPÄISCHE PATENTSCHRIFT

(11) **EP 1 127 568 B1**
(45) Veröffentlichungstag und Bekanntmachung des Hinweises auf die Patenterteilung: **24.01.2007**
(21) Anmeldenummer: 01104074.8
(22) Anmeldetag: 21.02.2001
(51) Int. Cl.: A61Q 17/04, A61K 8/49, A61K 8/37

(54) **Kosmetische und dermatologische Lichtschutzformulierungen mit einem Gehalt an Benzotriazolderivaten und Alkylnapthalaten**
Cosmetic and dermatological light protective formulations containing benzotriazole derivatives and alkylnaphthalates
Formulations photoprotectrices cosmétiques et dermatologiques contenant des dérivés de benzotriazole et naphthalates d'alkyle

(30) Priorität: 25.02.2000 DE 10008896
(43) Veröffentlichungstag der Anmeldung: 29.08.2001
(73) Patentinhaber: Beiersdorf AG, 20253 Hamburg (DE)
(72) Erfinder: Gers-Barlag, Heinrich, Dr., 25495 Kummerfeld (DE); Wendel, Volker, Dr., 20255 Hamburg (DE); Grundt, Wiebke, 21244 Buchholz (DE)

(56) Entgegenhaltungen:
- EP-A- 0 943 321
- FR-A- 2 801 206
- US-A- 5 993 789
- BONDA C ET AL: "A NEW PHOTOSTABILIZER FOR FULL SPECTRUM SUNCREENS" COSMETICS & TOILETRIES, WHEATON, IL, US, Bd. 115, Nr. 6, 2000, Seiten 37-45, XP001010090 ISSN: 0361-4387
- MÜLLER S. ET AL: 'Micronization as a Tool in the Development of Innovative UV Filters' SÖFW

## Beschreibung

Die vorliegende Erfindung betrifft kosmetische und dermatologische Lichtschutzzubereitungen, insbesondere hautpflegende kosmetische und dermatologische Lichtschutzzubereitungen.

Die schädigende Wirkung des ultravioletten Teils der Sonnenstrahlung auf die Haut ist allgemein bekannt. In Abhängigkeit von ihrer jeweiligen Wellenlänge haben die Strahlen verschiedene Wirkungen auf das Organ Haut: Die sogenannte UV-C-Strahlung mit einer Wellenlänge, die kleiner als 290 nm ist, wird von der Ozonschicht in der Erdatmosphäre absorbiert und hat daher keine physiologische Bedeutung. Dagegen verursachen Strahlen im Bereich zwischen 290 nm und 320 nm, dem sogenannten UV-B-Bereich, ein Erythem, einen einfachen Sonnenbrand oder sogar mehr oder weniger starke Verbrennungen. Als ein Maximum der Erythemwirksamkeit des Sonnenlichtes wird der engere Bereich um 308 nm angegeben.

Zum Schutz gegen UV-B-Strahlung sind zahlreiche Verbindungen bekannt, bei denen es sich beispielsweise um Derivate des 3-Benzylidencamphers, der 4-Aminobenzoesäure, der Zimtsäure, der Salicylsäure, des Benzophenons, des 2-Phenylbenzimidazols sowie des s-Triazins handelt.

Man hat lange Zeit fälschlicherweise angenommen, daß die langwellige UV-A-Strahlung mit einer Wellenlänge zwischen 320 nm und 400 nm nur eine vemachlässigbare biologische Wirkung aufweist. Inzwischen¹ ist allerdings durch zahlreiche Studien belegt, daß UV-A-Strahlung im Hinblick auf die Auslösung photodynamischer, speziell phototoxischer Reaktionen und chronischer Veränderungen der Haut weitaus gefährlicher als UV-B-Strahlung ist.

Auch kann der schädigende Einfluß der UV-B-Strahlung durch UV-A-Strahlung noch verstärkt werden.

So ist es u.a. erwiesen, daß selbst die UV-A-Strahlung unter ganz normalen Alltagsbedingungen ausreicht, um innerhalb kurzer Zeit die Collagen- und Elastinfasem zu schädigen, die für die Struktur und Festigkeit der Haut von wesentlicher Bedeutung sind. Hierdurch kommt es zu chronischen lichtbedingten Hautveränderungen - die Haut "altert" vorzeitig. Zum klinischen Erscheinungsbild der durch Licht gealterten Haut gehören beispielsweise Falten und Fältchen sowie ein unregelmäßiges, zerfurchtes Relief. Femer können die von lichtbedingter Hautalterung betroffenen Partien eine unregelmäßige Pigmentierung aufweisen. Auch die Bildung von braunen Flecken, Keratosen und sogar Karzinomen bzw. malignen Melanomen ist möglich. Eine durch die alltägliche UV-Belastung vorzeitig gealterte Haut zeichnet sich außerdem durch eine geringere Aktivität der Langerhanszellen und eine leichte, chronische Entzündung aus.

Etwa 90 % der auf die Erde gelangenden ultravioletten Strahlung besteht aus UV-A-Strahlen. Während die UV-B-Strahlung in Abhängigkeit von zahlreichen Faktoren stark variiert (z.B. Jahres- und Tageszeit oder Breitengrad), bleibt die UV-A-Strahlung unabhängig von jahres- und tageszeitlichen oder geographischen Faktoren Tag für Tag relativ konstant. Gleichzeitig dringt der überwiegende Teil der UV-A-Strahlung in die lebende Epidermis ein, während etwa 70 % der UV-B-Strahlen von der Homschicht zurückgehalten werden.

Es ist daher von grundsätzlicher Wichtigkeit, daß kosmetische und dermatologische Lichtschutzzubereitungen sowohl gegen UV-B- als auch gegen UV-A-Strahlung ausreichenden Schutz bieten.

Im allgemeinen ist das Lichtabsorptionsverhalten von Lichtschutzfiltersubstanzen sehr gut bekannt und dokumentiert, zumal in den meisten Industrieländern Positivlisten für den Einsatz solcher Substanzen existieren, welche recht strenge Maßstäbe an die Dokumentation anlegen.

Die Einsatzkonzentration bekannter Lichtschutzfiltersubstanzen ist allerdings häufig gerade in Kombination mit anderen als Festkörper vorliegenden Substanzen begrenzt. Es bereitet daher gewisse formulierungstechnische Schwierigkeiten, höhere Lichtschutzfaktoren bzw. UV-A-Schutzleistung zu erzielen.

Vorteilhafte Lichtschutzfilter sind solche, die sich durch das Strukturmotiv des Benzotriazols 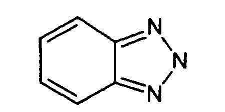 auszeichnen.

Ein vorteilhaftes Benzotriazolderivat ist das 2,2'-Methylen-bis-(6-(2H-benzotriazol-2-yl)-4-(1,1,3,3-tetramethylbutyl)-phenol), welches durch die chemische Strukturformel 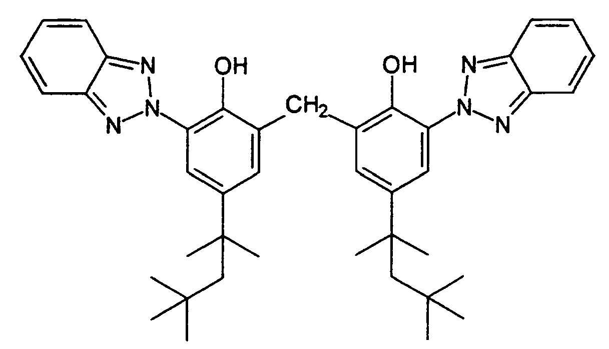 gekennzeichnet ist (INCI: Bisoctyltriazol). Es ist unter der Handelsbezeichnung Tinosorb® M bei der CIBA-Chemikalien GmbH erhältlich und zeichnet sich durch gute UV-Absorptionseigenschaften aus. Nachteilig an dieser Substanz ist die Eigenart, auf der Haut unmerklich dünne Filme zu bilden, die taktile Unannehmlichkeiten aufweisen.

Ein weiterer Nachteil liegt darin, daß solche Benzotriazolderivate nicht oder nur unzureichend in üblichen Ölkomponenten löslich sind. In bekannten Lösungsmitteln lassen sich nur bis zu maximal 15 Gew.-% dieser Verbindungen lösen, was im Regelfall einer Konzentration von etwa 1 bis 1,5 Gew.-% gelöster (= aktiver) Filtersubstanz in der gesamten kosmetischen oder dermatologischen Zubereitung entspricht.

Ein Nachteil des Standes der Technik ist dementsprechend, daß mit diesen Filtersubstanzen in der Regel nur vergleichsweise niedrige Lichtschutzfaktoren erreicht werden konnten, da ihre Löslichkeit oder Dispergierbarkeit in den Formulierungen zu gering ist, d. h. sie lassen sich nicht oder nur schwer in befriedigender Weise in solche Formulierungen einarbeiten.

Selbst wenn grundsätzlich auch bei begrenzter Löslichkeit ein gewisser UV-Schutz erreicht werden kann, tritt häufig ein anderes Problem auf, die Rekristallisation.

Insbesondere schlecht lösliche Substanzen rekristallisieren vergleichsweise schnell, was durch Temperaturschwankungen oder andere Einflüsse hervorgerufen werden kann. Unkontrollierte Rekristallisation eines wesentlichen Zubereitungsbestandteiles wie eines UV-Filters hat aber äußerst nachteilige Einwirkungen auf die Eigenschaften der gegebenen Zubereitung und, nicht zuletzt, auf den angestrebten Lichtschutz.

Es war Aufgabe der vorliegenden Erfindung, auf einfache Weise zu Zubereitungen zu gelangen, welche sich durch einen erhöhten Gehalt an bestimmten Benzotriazolderivaten und einen demensprechend hohen Lichtschutzfaktor auszeichnen.

Es war indes überraschend und für den Fachmann nicht vorauszusehen, daß lichtschutzwirksame kosmetische Formulierungen, dadurch gekennzeichnet, dass sie
(a) eine oder mehrere UV-Filtersubstanzen, gewählt aus der Gruppe der Benzotriazolderivate, welche sich durch die Struktur 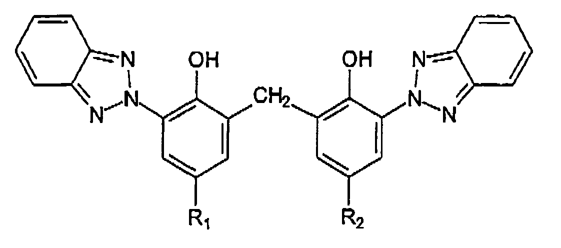 auszeichnen, wobei R₁ und R₂ unabhängig voneinander gewählt werden aus der Gruppe der verzweigten oder unverzweigten C₁-C₁₈-Alkylreste,
   und
(b) ein oder mehrere DialkyInaphthalate, welche sich durch die Strukturformel 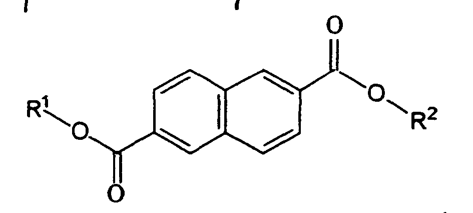 auszeichnen, worin R¹ und R² unabhängig voneinander gewählt werden aus der Gruppe der verzweigten und unverzweigten Alkylgruppen mit 6 bis 24 Kohlenstoffatomen enthalten, den Nachteilen des Standes der Technik abhelfen.

In einer weiteren besonders vorteilhaften Ausführungsform betrifft die vorliegende Erfindung die Verwendung von einem oder mehreren Dialkylnaphthalaten, welche sich durch die Strukturformel 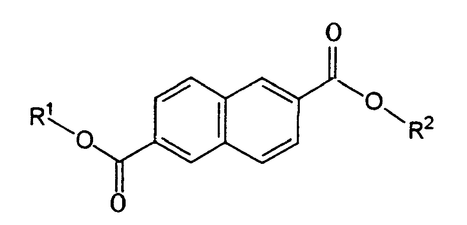 auszeichnen, worin R¹ und R² unabhängig voneinander gewählt werden aus der Gruppe der verzweigten und unverzweigten Alkylgruppen mit 6 bis 24 Kohlenstoffatomen, zur Verbesserung der Einarbeitbarkeit von Benzotriazolderivaten in
(a) entweder einer isolierten Ölkomponente oder
(b) in mindestens einer Ölphase eines dispersen Zwei- oder Mehrphasensystems, welches zusätzlich eine oder mehrere Wasserphasen enthalten kann.

Es wird, zumal bei ganz besonders schwerlöslichen Benzotriazolderivaten, davon ausgegangen, daß das oder die Dialkylnaphthalate die Eigenschaft an den Tag legen, solche Benzotriazolderivate besser feindispers
(a) entweder einer isolierten Ölkomponente oder
(b) in mindestens einer Ölphase eines dispersen Zwei- oder Mehrphasensystem, welches zusätzlich eine oder mehrere Wasserphasen enthalten kann.
zu verteilen als die Komponenten das Standes der Technik dies vermochten oder nahelegten.

Es ist erfindungsgemäß bevorzugt, die dispersen Zwei- oder Mehrphasensysteme, welche neben einer oder mehrerer Ölphasen zusätzlich eine oder mehrere Wasserphasen enthalten können, in Form kosmetischer oder dermatologischer Emulsionen - beispielsweise vom Typ W/O, O/W, W/O/W oder O/W/O - auszugestalten. Solche Emulsionen können vorzugsweise auch eine Mikroemulsion, eine Pickering-Emulsion oder eine sprühbare Emulsion sein. Es kann aber auch von Vorteil sein, wenn die erfindungsgemäßen Zubereitungen eine Lösung, eine Hydrodispersion, ein Aerosol, einen Schaum oder auch einen Stift darstellen.

Vorteilhaft im Sinne der vorliegenden Erfindung sind Dialkylnaphthalate, für die R¹ und/oder R² verzweigte Alkylgruppen mit 6 bis 10 Kohlenstoffatomen darstellen. Ganz besonders bevorzugt ist Diethylhexylnaphthalat.

Das bevorzugte Benzotriazolderivat ist das 2,2'-Methylen-bis-(6-(2H-benzotriazol-2-yl)-4-(1,1,3,3-tetramethylbutyl)-phenol), welches durch die chemische Strukturformel 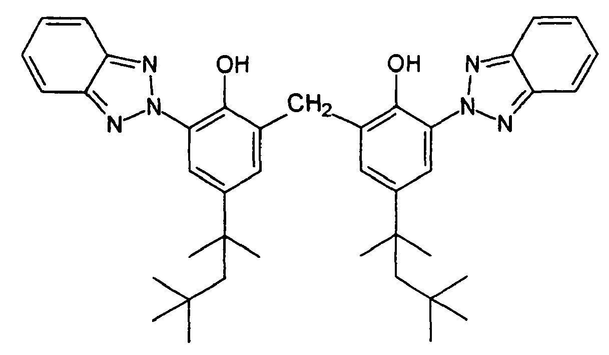 gekennzeichnet ist

Die Gesamtmenge an einem oder mehreren Benzotriazolderivaten, insbesondere von 2,2'-ethylen-bis-(6-(2H-benzotriazol-2-yl)-4-(1,1,3,3-tetramethylbutyl)-phenol), in den fertigen kosmetischen oder dermatologischen Zubereitungen wird vorteilhaft aus dem Bereich von 0,1 bis 15,0 Gew.-%, bevorzugt 0,5 bis 10,0 Gew.-% gewählt, bezogen auf das Gesamtgewicht der Zubereitungen.

Die erfindungsgemäßen kosmetischen oder dermatologischen Lichtschutzformulierungen können wie üblich zusammengesetzt sein und dem kosmetischen oder dermatologischen Lichtschutz, ferner zur Behandlung, Pflege und Reinigung der Haut und/oder der Haare und als Schminkprodukt in der dekorativen Kosmetik dienen.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Ein zusätzlicher Gehalt an Antioxidantien ist im allgemeinen bevorzugt. Erfindungsgemäß können als günstige Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Camosin, D-Carnosin, L-Camosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linotensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Camosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Ölphase der Formulierungen, welche die erfindungsgemäße Stoffkombination enthalten, wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z.B. Caprylic/Caprictriglycerid, Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkemöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z.B. Jojobaöl.

Femer kann die Ölphase vorteilhaft gewählt werden aus der Gruppe der Dialkylether, vorteilhaft ist z. B. Dicaprylylether.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Caprylat/Caprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Erfindungsgemäß vorteilhaft sind ferner z. B. natürliche Wachse tierischen und pflanzlichen Ursprungs, wie beispielsweise Bienenwachs, Chinawachs, Hummelwachs und andere Insektenwachse sowie Sheabutter.

Die wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Auch Moisturizer können bevorzugt verwendet werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hautoberfläche die Feuchtigkeitsabgabe der Homschicht (auch transepidermal water loss (TEWL) genannt) zu reduzieren und/oder die Hydratation der Homschicht positiv zu beeinflussen.

Vorteilhafte Moisturizer im Sinne der vorliegenden Erfindung sind beispielsweise Glycerin, Milchsäure, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registratumummer 178463-23-5 abgelegt und z. B. unter der Bezeichnung Fucogel®1000 von der Gesellschaft SOLABIA S.A. erhältlich ist.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine weitere UV-A-Filtersubstanz und/oder mindestens eine weitere UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes gewöhnlich UV-A- bzw. UV-B-Filtersubstanzen eingearbeitet

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft enthalten erfindungsgemäße Zubereitungen Substanzen, die UV-Strahlung im UV-A- und/oder UV-B-Bereich absorbieren, wobei die Gesamtmenge der Filtersubstanzen z. B. 0,1 Gew.-% bis 30 Gew.-%, vorzugsweise 0,5 bis 20 Gew.-%, insbesondere 1,0 bis 15,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UV-B-Filtersubstanzen, können diese öllöslich oder wasserlöslich sein. Erfindungsgemäß vorteilhafte öllösliche UV-B-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester,
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- hinsichtlich der C₃-Achse des Triazingrundkörpers symmetrisch Triazinderivate, vorzugsweise 4,4',4"-(1,3,5-Triazin-2,4,6-triyltriimino)-tris-benzoesäure-tris(2-ethylhexylester) [INCI: Octyl Triazone], welches von der BASF Aktiengesellschaft unter der Warenbezeichnung UVINUL® T 150 vertrieben wird,
- sowie an Polymere gebundene UV-Filter.

Vorteilhafte wasserlösliche UV-B-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bomylidenmethyl)benzolsulfonsäure, 2-Methyl-5-(2-oxo-3-bomylidenmethyl)sulfonsäure und ihre Salze.

Die Liste der genannten UV-B-Filter, die in den erfindungsgemäßen Zubereitungen verwendet werden können, soll selbstverständlich nicht limitierend sein.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Es kann auch von Vorteil sein, in erfindungsgemäßen Zubereitungen UV-A-Filter zu verwenden, die bisher üblicherweise in kosmetischen Zubereitungen enthalten sind. Bei diesen Substanzen handelt es sich vorzugsweise um Derivate des Dibenzoylmethans, insbesondere um 1-(4'-tert. Butylphenyl)-3-(4'-methoxyphenyl)propan-1,3-dion und um 1-Phenyl-3-(4'-isopropylphenyl)propan-1,3-dion.

Ferner kann von Vorteil sein, zusätzlich zu den erfindungswesentlichen Bestandteilen weitere unsymmetrisch substituierte s-Triazinderivate zuzufügen, beispielsweise solche, wie sie in der EP-A-570 838 beschrieben werden, deren chemische Struktur durch die generische Formel 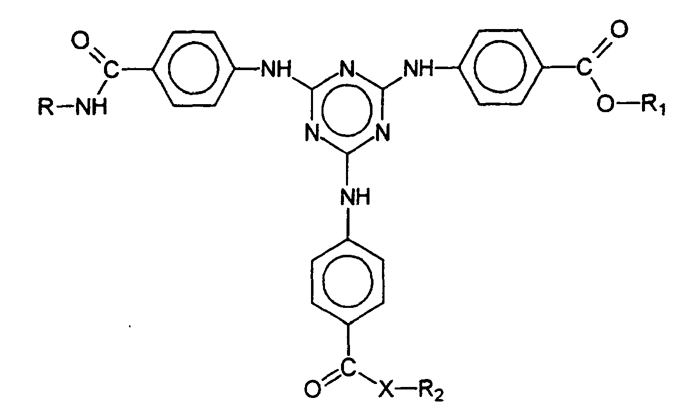 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 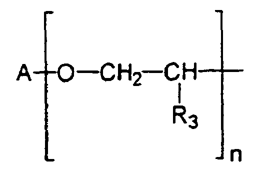 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄-Alkylgruppen, oder ein Wasserstoffatom, ein Alkalimetallatom, eine Ammoniumgruppe oder eine Gruppe der Formel 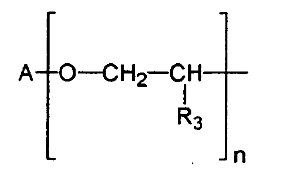 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄-Alkylgruppen,
R₃ ein Wasserstoffatom oder eine Methylgruppe darstellt,
n eine Zahl von 1 bis 10 darstellt, wenn X ein Sauerstoffatom darstellt.

Insbesondere vorteilhaft sind solche unsymmetrisch substituierte s-Triazine, aus der Gruppe der Substanzen gewählt, welche in der EP-A-775 698 beschrieben werden: 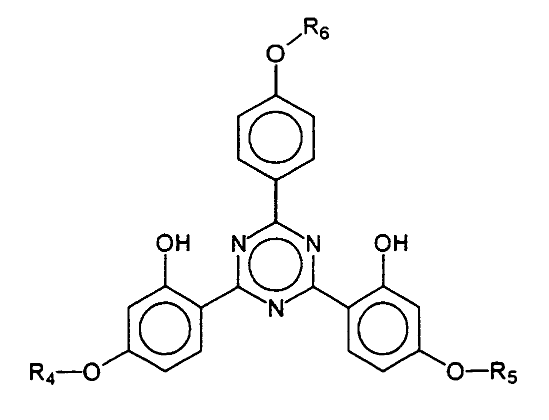 und/oder

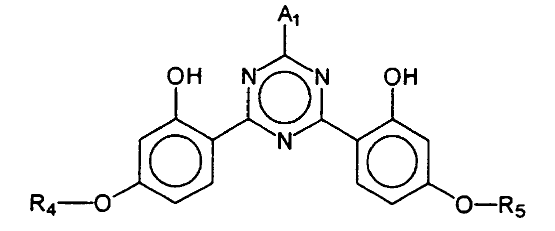

Alle in dieser Schrift erwähnten Bis-Resorcinyltriazine, seien sie durch generische oder durch konkrete Formeln offenbart, sind vorteilhaft im Sinne der vorliegenden Erfindung. Ganz besonders vorteilhaft werden R₄ und R₅ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

A₁ stellt vorteilhaft einen substituierten homo- oder heterocyclischen aromatischen Fünfring oder Sechsring dar.

Ganz besonders vorteilhaft sind folgende Verbindungen: 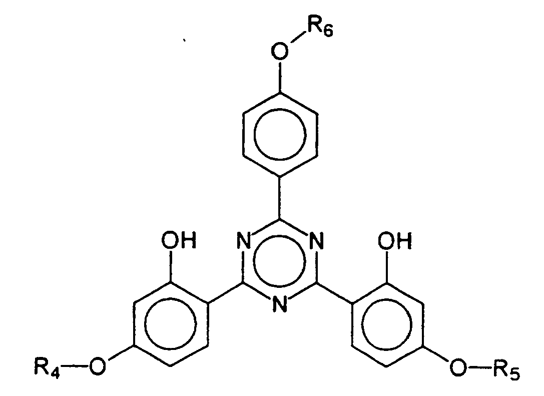 wobei R₆ ein Wasserstoffatom oder eine verzweigte oder unverzweigte Alkylgruppe mit 1 bis 10 Kohlenstoffatomen darstellt, insbesondere das 2,4-Bis-{[4-(2-ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich und durch folgende Struktur gekennzeichnet ist:

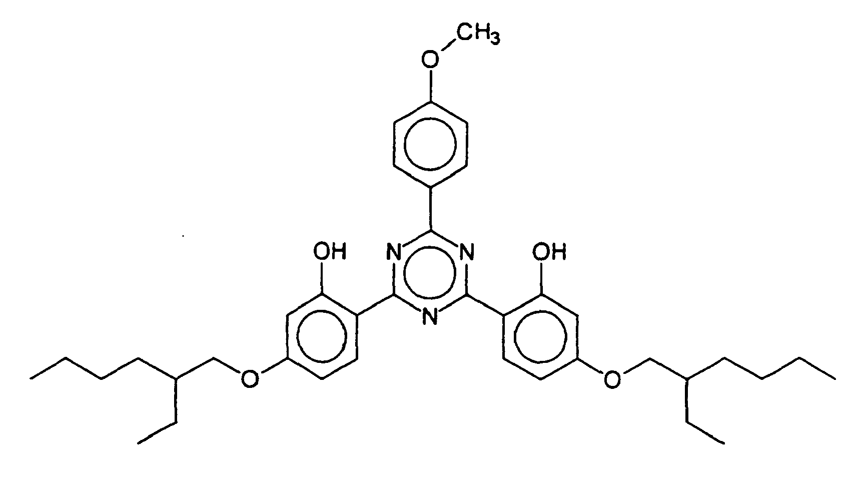

Es ist auch gegebenenfalls vorteilhaft, Zubereitungen gemäß der Erfindung mit einem Gehalt an 2,4-Bis-{[4-(3-sulfonato)-2-hydroxy-propyloxy)-2-hydroxy]-phenyl}-6-(4-methoxyphenyl)-1,3,5-triazin Natriumsalz zu versehen, welches durch folgende Struktur gekennzeichnet ist:

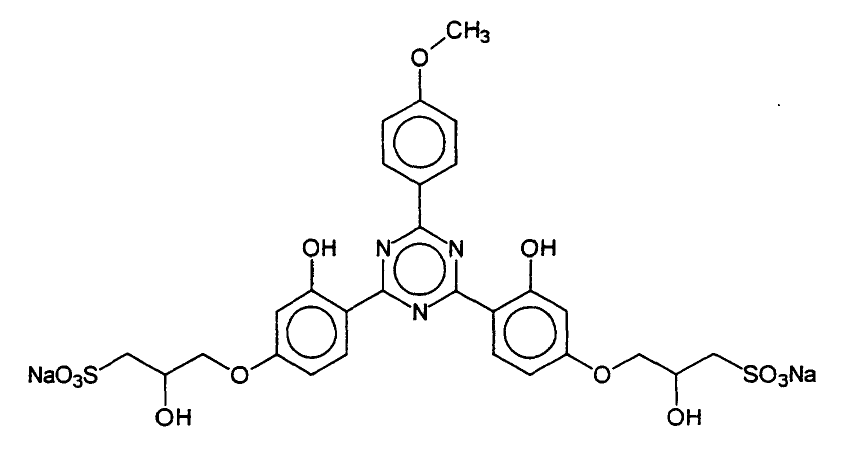

Es ist auch gegebenenfalls vorteilhaft, Zubereitungen gemäß der Erfindung mit einem Gehalt an 2,4-Bis-{[4-(3-(2-propyloxy)-2-hydroxy-propyloxy)-2-hydroxy]-phenyl}-6-(4-methoxyphenyl)-1,3,5-triazin zu versehen, welches durch folgende Struktur gekennzeichnet ist:

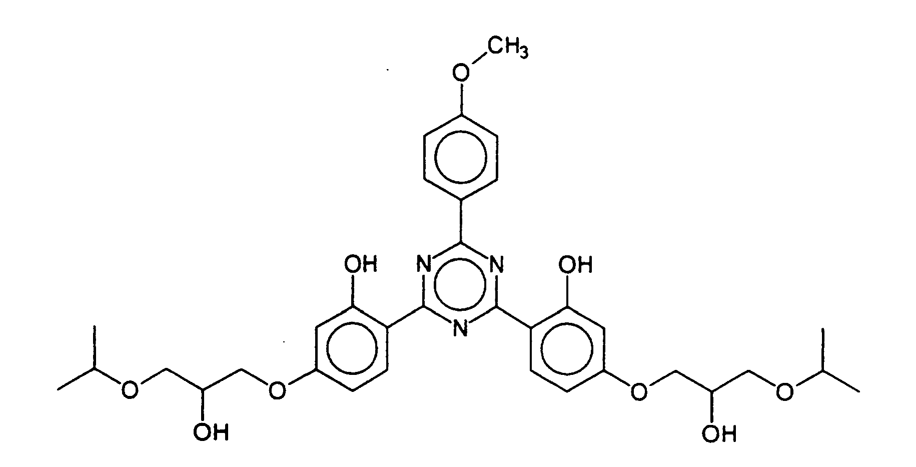

Es ist auch gegebenenfalls vorteilhaft, Zubereitungen gemäß der Erfindung mit einem Gehalt an 2,4-Bis-{[4-(2-ethyl-hexyloxy)-2-hydroxy]-phenyI}-6-[4-(2-methoxyethyl-carboxyl)-phenylamino]-1,3,5-triazin zu versehen, welches durch folgende Struktur gekennzeichnet ist:

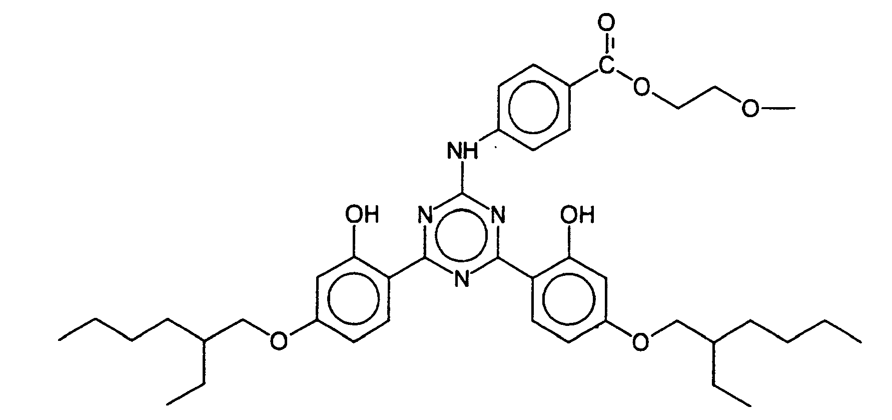

Es ist auch gegebenenfalls vorteilhaft, Zubereitungen gemäß der Erfindung mit einem Gehalt an 2,4-Bis-{[4-(3-(2-propyloxy)-2-hydroxy-propyloxy)-2-hydroxy]-phenyl}-6-[4-(ethylcarboxyl)-phenylamino]-1,3,5-triazin zu versehen, welches durch folgende Struktur gekennzeichnet ist:

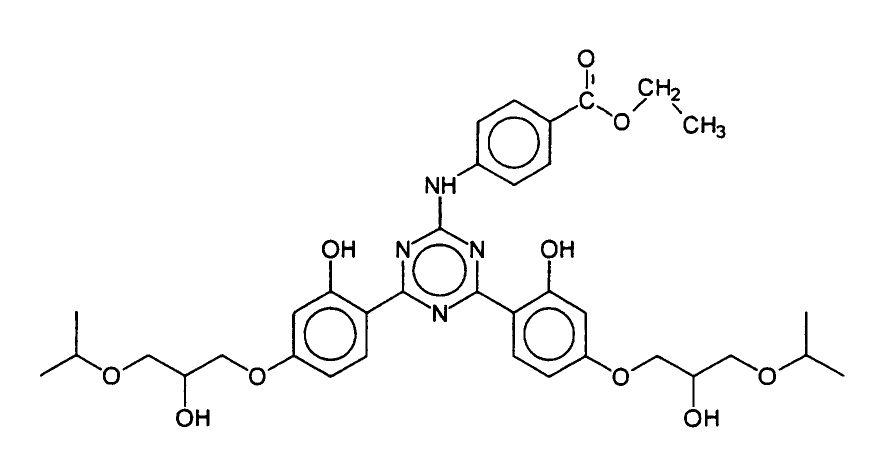

Es ist auch gegebenenfalls vorteilhaft, Zubereitungen gemäß der Erfindung mit einem Gehalt an 2,4-Bis-{[4-(2-ethyl-hexyloxy)-2-hydroxy]-phenyl}-6-(1-methyl-pyrrol-2-yl)-1,3,5-triazin zu versehen, welches durch folgende Struktur gekennzeichnet ist:

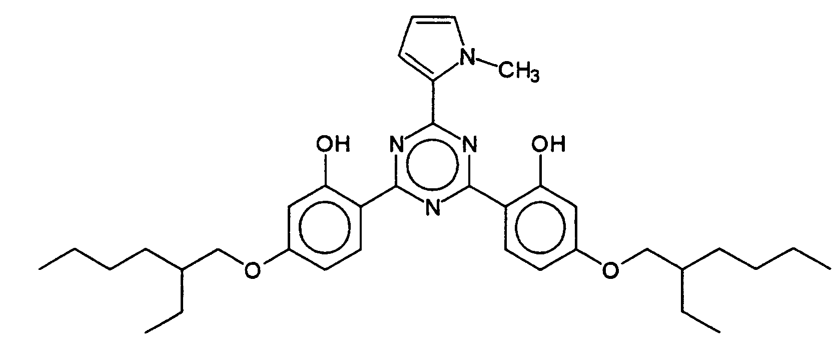

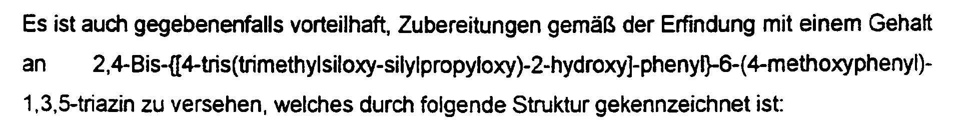

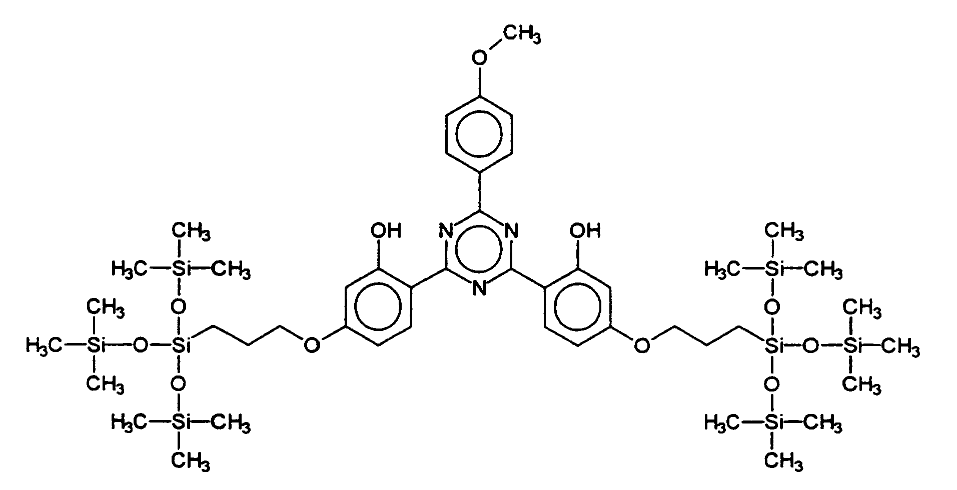

Es ist auch gegebenenfalls vorteilhaft, Zubereitungen gemäß der Erfindung mit einem Gehalt an 2,4-Bis-{[4-(2-methylpropenyloxy)-2-hydroxy]-phenyl}-6-(4-methoxyphenyl)-1,3,5-triazin zu versehen, welches durch folgende Struktur gekennzeichnet ist:

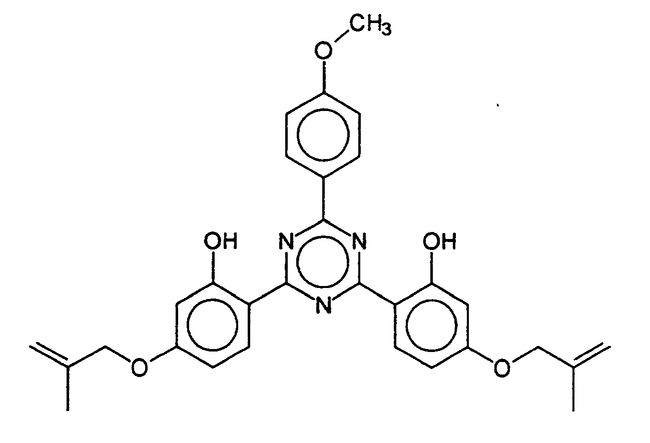

Es ist auch gegebenenfalls vorteilhaft, Zubereitungen gemäß der Erfindung mit einem Gehalt an 2,4-Bis-{[4-(1',1',1',3',5',5',5'-Heptamethylsiloxy-2-methyl-propyloxy)-2-hydroxy]-phenyl)-6-(4-methoxyphenyl)-1,3,5-triazin zu versehen, welches durch folgende Struktur gekennzeichnet ist:

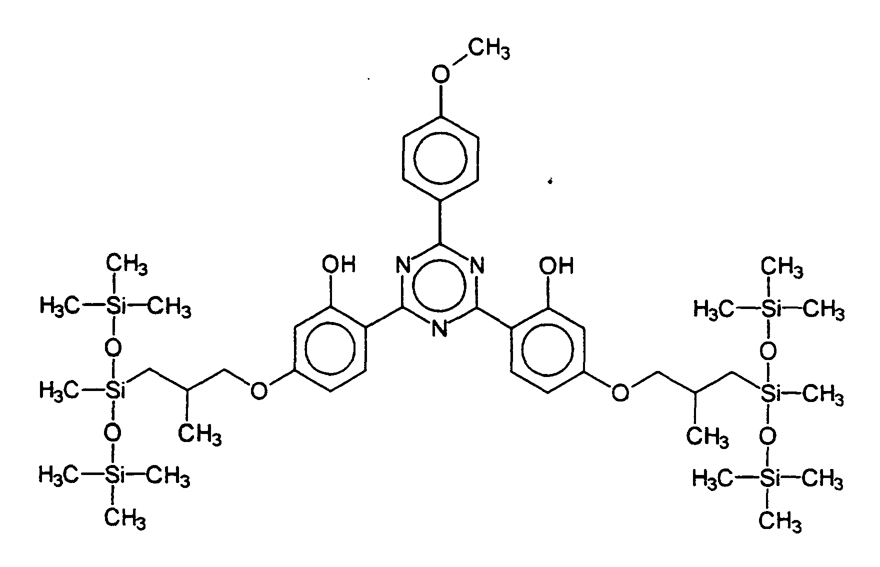

Es ist auch gegebenenfalls vorteilhaft, Zubereitungen gemäß der Erfindung mit einem Gehalt eines asymmetrisch substituierten s-Triazins zu versehen, dessen chemische Struktur durch die Formel 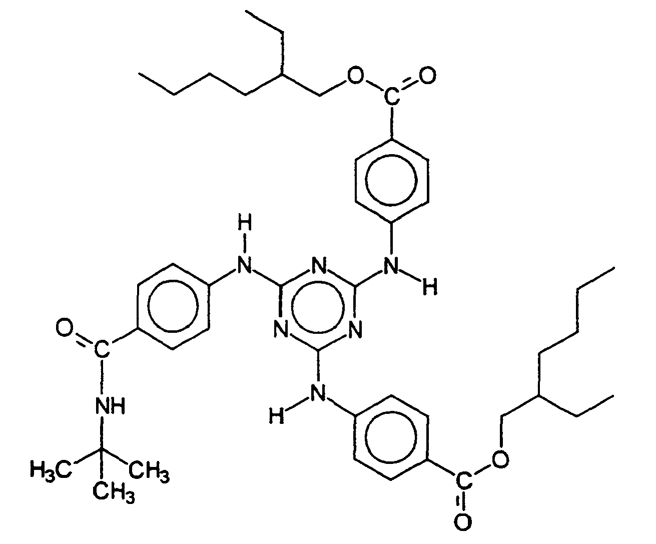 wiedergegeben wird, welche im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Das oder die symmetrisch oder unsymmetrisch substituierten s-Triazinderivate werden, falls ihre Gegenwart erwünscht ist, vorteilhaft in die Ölphase der kosmetischen oder dermatologischen Formulierungen eingearbeitet.

Weitere vorteilhafte UV-A-Filtersubstanzen sind die Phenylen-1,4-bis-(2-benzimidazyl)-3,3'-5,5'-tetrasulfonsäure: 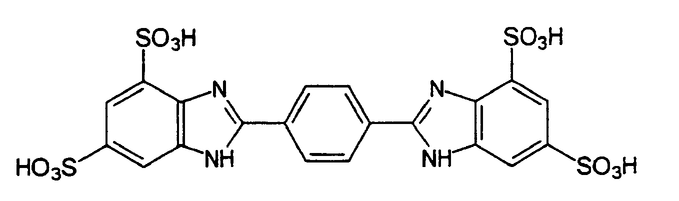 und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz: 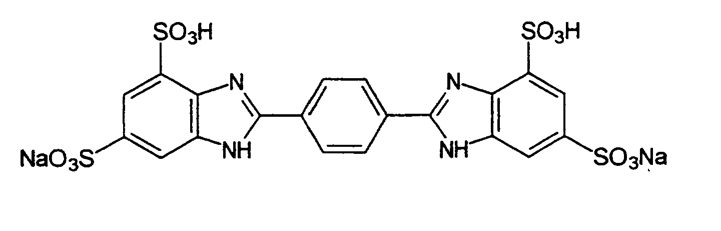 sowie das 1,4-Di(2-oxo-10-sulfo-3-bomylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bomylidenmethyl-10-sulfonsäure) bezeichnet wird und sich durch die folgende Struktur auszeichnet:

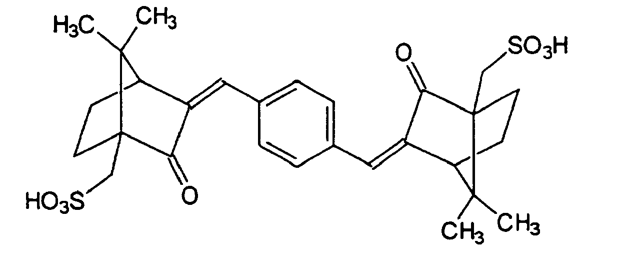

Auch Zubereitungen, die UV-A-Filter enthalten, sind Gegenstand der Erfindung. Es können die für die UV-B-Kombination verwendeten Mengen eingesetzt werden.

Femer kann es gegebenenfalls von Vorteil sein, erfindungsgemäß weitere UV-A- und/oder UV-B-Filter in kosmetische oder dermatologische Zubereitungen einzuarbeiten, beispielsweise bestimmte Salicylsäurederivate wie 4-lsopropylbenzylsalicylat, 2-Ethylhexylsalicylat (= Octylsalicylat), Homomenthylsalicylat.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 erhältlich ist und sich durch folgende Struktur auszeichnet:

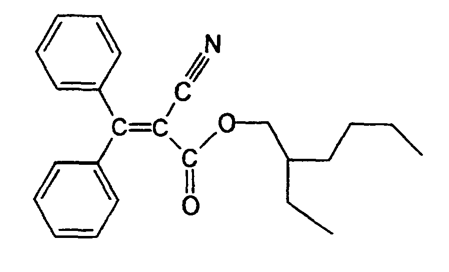

Die Liste der genannten UV-Filter, die im Sinne der vorliegenden Erfindung eingesetzt werden können, soll selbstverständlich nicht limitierend sein.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

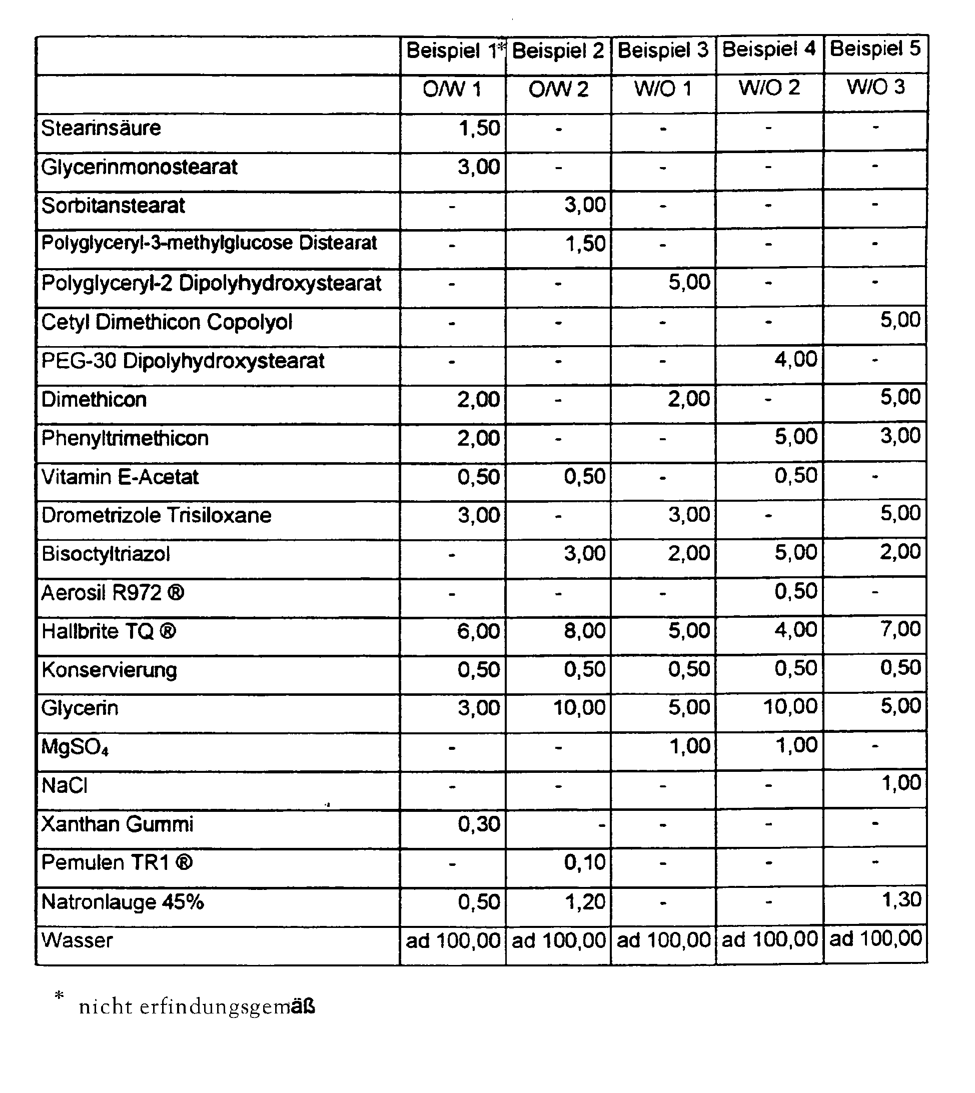

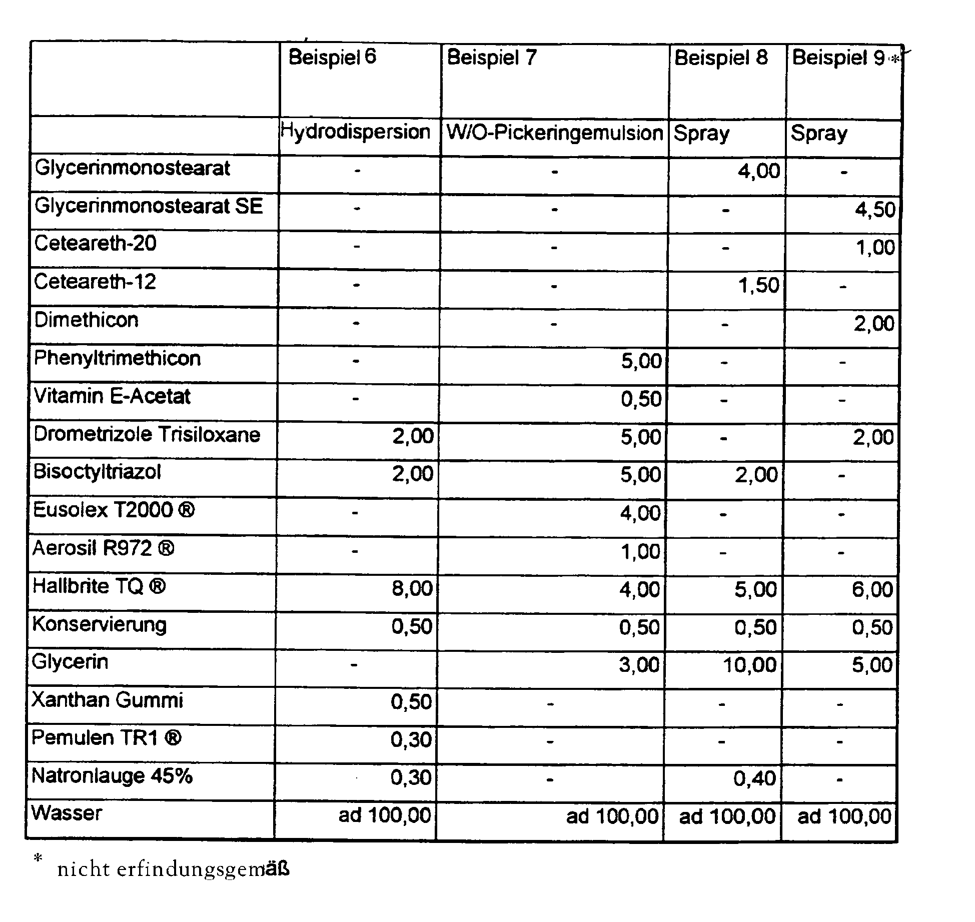

## Patentansprüche

1. Lichtschutzwirksame kosmetische Formulierung, **dadurch gekennzeichnet, dass** sie
a) eine oder mehrere UV-Filtersubstanzen, gewählt aus der Gruppe der Benzotriazol derivate, welche sich durch die Struktur 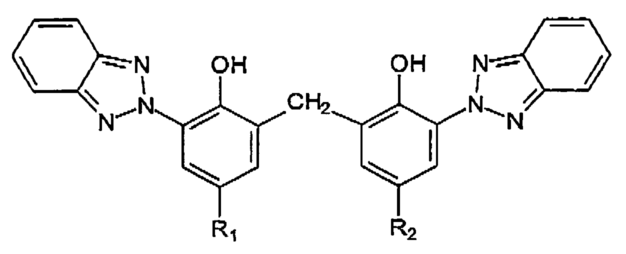 auszeichnen, wobei R₁ und R₂ unabhängig voneinander gewählt werden aus der Gruppe der verzweigten oder unverzweigten C₁-C₁₈-Arlkylreste,
und
b) ein oder mehrere Dialkylnaphthalate, welche sich durch die Strukturformel 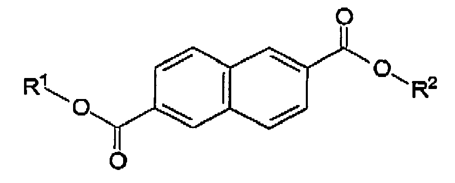 auszeichnen, worin R¹ und R² unabhängig voneinander gewählt werden aus der Gruppe der verzweigten und unverzweigten Alkylgruppen mit 6 bis 24 Kohlenstoffatomen,
enthält.

2. Zubereitung nach Anspruch 1, **dadurch gekennzeichnet, dass** als Dialkylnaphthalat Diethylhexylnaphthalat gewählt wird.

3. Zubereitung nach einem der Ansprüche 1 oder 2, **dadurch gekennzeichnet**, als Benzotriazol das 2,2'-Methylen-bis-(6-(2H-benzotriazol-2-yl)-4-(1,1,3,3-tetramethylbutyl)-phenol) gewählt wird, welches durch die chemische Strukturformel 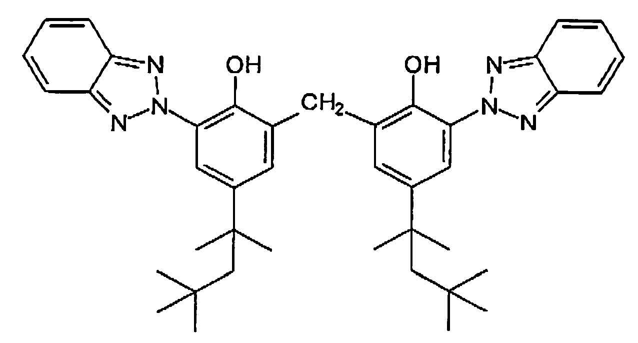 gekennzeichnet ist.

4. Verwendung von einem oder mehreren Dialkylnaphthalaten, welche sich durch die Strukturformel 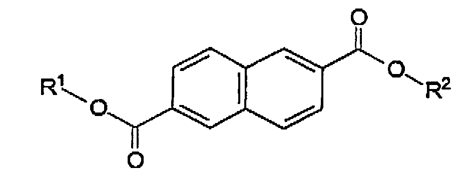 auszeichnen, worin R¹ und R² unabhängig voneinander gewählt werden aus der Gruppe der verzweigten und unverzweigten Alkylgruppen mit 6 bis 24 Kohlenstoffatomen, zur Verbesserung der Einarbeitbarkeit von Benzotriazolderivaten in
a) entweder eine isolierte Ölkomponente oder
b) in mindestens eine Ölphase eines dispersen Zwei- oder Mehrphasensystems, welches zusätzlich eine oder mehrere Wasserphasen enthalten kann.

## Claims

1. Cosmetic formulation effective for light protection, **characterized in that** it comprises
a) one or more UV filter substances selected from the group of benzotriazole derivatives having the structure 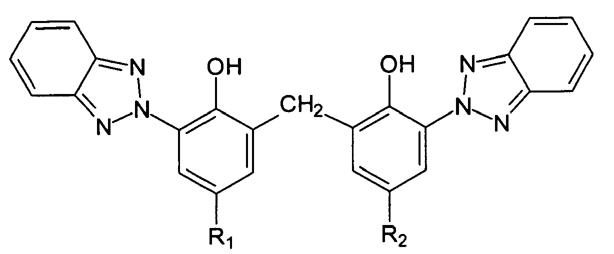 where R₁ and R₂ are, independently of one another, selected from the group of branched or unbranched C₁-C₁₈-alkyl radicals,
and
b) one or more dialkyl naphthalates having the structural formula 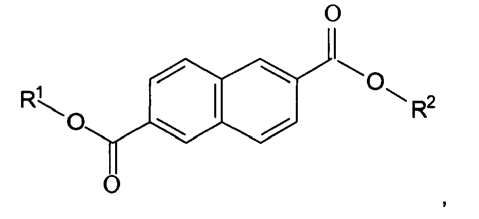 in which R¹ and R² are, independently of one another, selected from the group of branched and unbranched alkyl groups having 6 to 24 carbon atoms.

2. Preparation according to claim 1, **characterized in that** the chosen dialkyl naphthalate is diethylhexyl naphthalate.

3. Preparation according to either of claims 1 or 2, **characterized in that** the chosen benzotriazole is 2,2'-methylenebis(6-(2H-benzotriazol-2-yl)-4-(1,1,3,3-tetramethylbutyl)phenol) which is **characterized by** the chemical structural formula

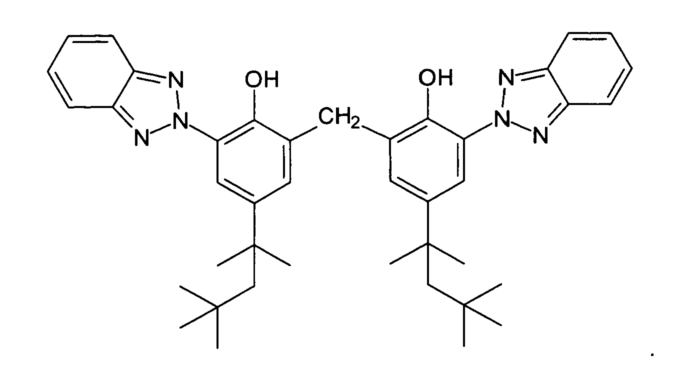

4. Use of one or more dialkyl naphthalates having the structural formula 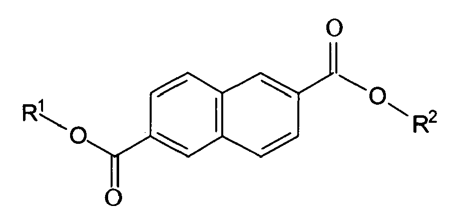 in which R¹ and R² are, independently of one another, selected from the group of branched and unbranched alkyl groups having 6 to 24 carbon atoms, for improving the incorporability of benzotriazole derivatives in
(a) either an isolated oil component or
(b) in at least one oil phase of a disperse two-phase or multiphase system which may additionally comprise one or more aqueous phases.

## Revendications

1. Formulation cosmétique active en protection contre la lumière, **caractérisée en ce qu'**elle contient
a) une ou plusieurs substances filtre des UV, choisies dans le groupe des dérivés du benzotriazole qui se caractérisent par la structure 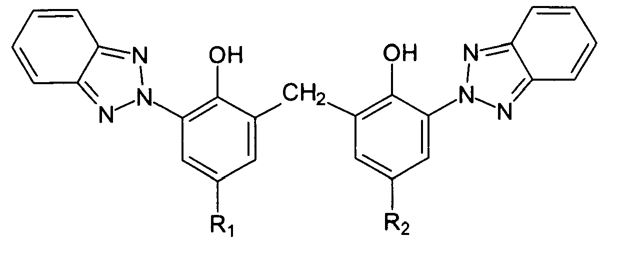 où R₁ et R₂ sont choisis indépendamment l'un de l'autre dans le groupe des radicaux C₁-C₁₈-alkyle ramifiés ou non ramifiés,
et
b) un ou plusieurs naphtalates de dialkyle, qui se caractérisent par la formule de structure 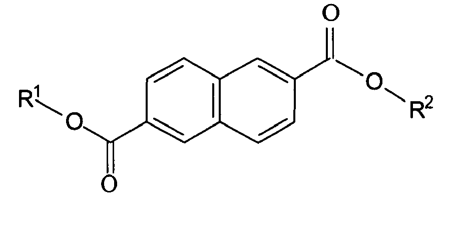
où R¹ et R² sont choisis indépendamment l'un de l'autre dans le groupe des groupements alkyle ramifiés et non ramifiés comprenant 6 à 24 atomes de carbone.

2. Préparation selon la revendication 1, **caractérisée en ce qu'**on choisit comme naphtalate de dialkyle le naphtalate de diéthylhexyle.

3. Préparation selon l'une quelconque des revendications 1 ou 2, **caractérisée en ce qu'**on choisit comme benzotriazole le 2,2'-méthylène-bis-(6-(2H-benzotriazol-2-yl)-4-(1,1,3,3-tétraméthylbutyl)-phénol), qui est **caractérisé par** la formule de structure chimique

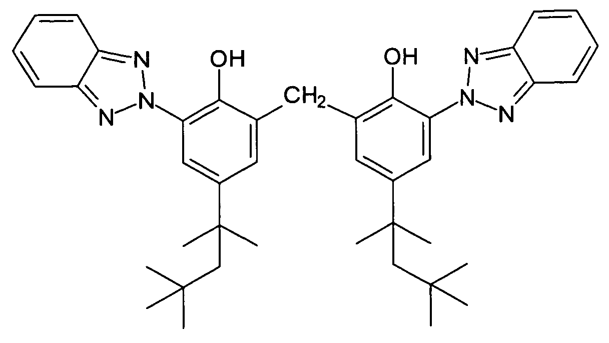

4. Utilisation d'un ou de plusieurs naphtalates de dialkyle, qui se caractérisent par la formule de structure, 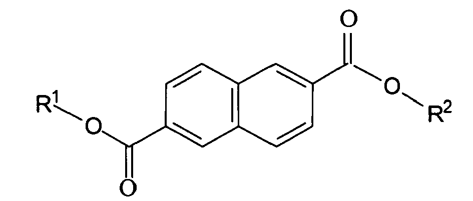 dans laquelle R¹ et R² sont choisis indépendamment l'un de l'autre dans le groupe des groupements alkyle ramifiés et non ramifiés comprenant 6 à 24 atomes de carbone, pour l'amélioration de l'aptitude à l'incorporation de dérivés de benzotriazole dans
a) un composé huileux isolé ou
b) au moins une phase huileuse d'un système dispersé à deux phases ou plus, qui peut en outre contenir une ou plusieurs phases aqueuses.
